# EUROPÄISCHE PATENTANMELDUNG

(11) **EP 1 324 254 A1**
(43) Veröffentlichungstag der Anmeldung: **02.07.2003**
(21) Anmeldenummer: 02027424.7
(22) Anmeldetag: 09.12.2002
(51) Int. Cl.: G06F 19/00

(54) **System zum Auffinden und Darstellen entscheidungsunterstützender Informationen in Archiven**

(30) Priorität: 21.12.2001 DE 10163470
(71) Anmelder: SIEMENS AKTIENGESELLSCHAFT, 80333 München (DE)
(72) Erfinder: Abraham-Fuchs, Klaus, 91058 Erlangen (DE)

(57) **Zusammenfassung**

System zum Auffinden und Darstellen entscheidungsunterstützender Informationen in elektronischen Patientenakten oder medizinischen Archiven, wobei ein kontextbasierter intelligenter Algorithmus aus den Eingaben des Arztes an einem lokalen computerbasierten Arbeitsplatz selbsttätig Schlüsselwörter ableitet und Patientenakten und/oder Archive nach diesen Schlüsselwörtern durchsucht, und wobei zu den Schlüsselwörtern relevante Informationen oder ein Link auf diese Informationen am lokalen computerbasierten Arbeitsplatz dargestellt werden.

## Beschreibung

Die Erfindung bezieht sich auf ein System zum Auffinden und Darstellen entscheidungsunterstützender Informationen in elektronischen Patientenakten oder medizinischen Archiven.

Die heutigen Gesundheitssysteme, speziell in den hochindustrialisierten Teilen der Welt, sind durch eine ausgeprägte Rollenverteilung und starke Spezialisierung der Ärzte geprägt. Dadurch wird medizinisch relevante Information an vielen verschiedenen Orten und zu unterschiedlichsten Zeitpunkten erhoben.

Es kommt daher häufig vor, dass einem gerade behandelnden Arzt die medizinische Information aus der Krankenhistorie des Patienten nicht vollständig zur Verfügung steht, die für eine aktuelle Diagnose oder Therapieentscheidung wichtig wäre.

Diese Situation wird durch die Einrichtung einer "Elektronischen Patientenakte" (EPR) mittels moderne I&K-Technologien in der Zukunft deutlich verbessert. Eine mögliche Implementierung einer EPR ist z. B., alle medizinisch relevanten Daten am Ort der Erhebung (Arztpraxis, Krankenhaus etc.) zu speichern, und diese Information durch Vernetzung mit einem zentralen Server anderen Berechtigten an jedem Ort und zu jeder Zeit verfügbar zu machen. Dies wird häufig als dezentralisierte "verteilte" EPR bezeichnet.

Dadurch ist zwar künftig theoretisch alle Information verfügbar, die der gerade behandelnde Arzt über die Krankenhistorie eines Patienten für eine optimale Therapieentscheidung benötigen würde. Die Informationsmenge wird jetzt aber viel zu groß sein, als dass der Arzt mit vertretbarem Zeitaufwand die verteilte elektronische Krankenakte bei jeder Entscheidungsfindung nach relevanter Information durchsuchen könnte.

Der Arzt ist daher bisher weitgehend auf die kooperative Information durch den Patienten selbst angewiesen. Dieser hat jedoch in der Regel nicht die nötige Fachkompetenz, um zu wissen, welche Information aus der Vergangenheit für den behandelnden Arzt wichtig wäre.

Aus der US 6,304,848 B1 ist zwar bereits eine Vorrichtung und ein Verfahren zur Verbesserung der Erstellung medizinischer Reporte beschrieben worden, bei welchem aus den Eingabedaten eines Arztes, gegebenenfalls unter Verwendung einer Schlüsselwortdatenbank, abgeleitet werden. Diese Schlüsselworte dienen einer besseren Strukturierung der Befunde und der medizinischen Aufzeichnungen für den Patienten sowie der Möglichkeit eines Zugriffs auf eine Wissensdatenbank, in der Behandlungsverfahren für entsprechende durch die Stichworte wiedergegebenen Krankheitsbilder enthalten sind. Es geht dabei aber nicht darum, die Datenlage hinsichtlich der Patientendaten zu verbessern und insbesondere nicht darum, zusätzlich zu den beim Arzt eingegebenen Patientendaten auch noch auf frühere Daten gerade dieses Patienten zurückzugreifen.

Entsprechend das gleiche gilt für Anordnungen zur Klassifizierung von Bildern und zur Erstellung von medizinischen Schlüsselwörtern, die mit den Bildern abgespeichert werden können, gemäß JP 10326279 A oder WO 01/37131 A2. Die bloße Erstellung von Schlüsselwörtern zu Bilddaten bietet für sich noch keine Zugriffsverbesserung auf elektronisch abgespeicherte Patientendaten.

Der Erfindung liegt daher die Aufgabe zugrunde, ein System zum Auffinden und Darstellen entscheidungsunterstützender Informationen in elektronischen Patientenakten oder medizinischen Archiven zu schaffen, das ohne großes Zutun des Arztes und ohne umständliche Eingaben und Suchhinweise auskommt.

Zur Lösung dieser Aufgabe ist erfindungsgemäß vorgesehen, dass ein kontextbasierter intelligenter Algorithmus aus den Eingaben des Arztes an einem lokalen computerbasierten Arbeitsplatz selbsttätig Schlüsselwörter ableitet und Patientenakten und/oder Archive nach diesen Schlüsselwörtern durchsucht, und dass zu den Schlüsselwörtern relevante Informationen oder ein Link auf diese Informationen am lokalen computerbasierten Arbeitsplatz dargestellt werden.

Das Wesen des erfindungsgemäßen Systems besteht dabei nicht darin, dass man mithilfe von Schlüsselwörtern Patientenakten oder Archive durchsucht, sondern in der automatischen Erstellung solcher Schlüsselwörter bei der Eingabe eines Krankheitsbefundes am lokalen computerbasierten Arbeitsplatz eines Arztes und der selbsttätigen, ohne Zutun und besonderen Anstoß des Arztes erfolgenden Suche in den häufig dezentralen Patientenakten des Patienten nach Eintragungen zu den so automatisch erstellten Schlüsselwörtern. Wenn der Arzt sich jedes Mal erst größere Gedanken machen muss, wo er am Besten suchen soll und was er denn an Vorinformationen zur besseren Befundung und Behandlung eines momentan von ihm untersuchten Patienten benötigt, vergeht viel zu viel Zeit und in der Arbeitshektik vergisst er möglicherweise auch die eine oder andere gezielte Anfrage. Auch das In-die-Wege-leiten einer solchen Anfrage ist wiederum ein gesonderter zeitaufwändiger Arbeitsgang, sodass solche Nachfragen angesichts der begrenzten Untersuchungszeit, die Ärzte bei der Vielzahl ihrer Patienten zur Verfügung steht, im Allgemeinen völlig unterbleiben. Der erfindungsgemäße Ansatz, dass die Schlüsselwörter automatisch aus den Eintragungen des Arztes erstellt und automatisch eine Suche in den Patientenakten oder im medizinischen Archiven gestartet wird, deren Ergebnis am lokalen computergestützten Arbeitsplatz angezeigt wird, kann für die angesprochene Problematik äußerst hilfreiche Ergebnisse liefern.

Dabei kann in Ausgestaltung der Erfindung vorgesehen sein, dass mit Hilfe eines medizinischen Tesaurus aus einem aus der Eingabe des Arztes abgeleiteten Stichwort mehrere verwandte Stichwörter abgeleitet werden, die zusätzlich zur Suche verwendet werden. Beispielsweise kann beim Auftreten des Wortes "Bauchschmerzen" in der Eingabe des Arztes selbsttätig ein weiterer Suchbegriff "Blinddarm" oder auch der lateinische Ausdruck "Appendix" durch einen solchen Tesaurus erzeugt werden, sodass auch unter diesen Suchwörtern recherchiert werden kann.

Dabei ist es in vielen Fällen besonders zweckmäßig, wenn ein aus der Eingabe des Arztes abgeleitetes Stichwort mit Hilfe eines solchen medizinischen Tesaurus in ein vordefiniertes verwandtes Suchwort übersetzt wird, um nicht unter allen möglichen vergleichbaren und zusammenhängenden Suchworten zu recherchieren, sondern um eine standardisierte Suche, beispielsweise immer unter Verwendung des lateinischen Ausdrucks, zu starten. Die Chance, unter diesem Ausdruck etwas zu finden, ist sehr viel größer, da in den medizinischen Lexika sicher unter den lateinischen Fachbegriffen sehr viel leichter Informationen gefunden werden. Entsprechendes gilt aber auch für Eintagungen anderer Ärzte in den elektronischen Patientenakten.

Anstelle der Suche unter dem Suchstichwort selbst, kann einem solchen Suchstichwort auch ein Verwaltungscode des Gesundheitswesens, wie z. B. ein sogenannter ICD-Code oder ein DRG-Code zugeordnet werden, um die Suche mit Hilfe dieses standardisierten Codes durchzuführen. Ein ICD-Code ist beispielsweise der Code betreffend die Abrechnungsziffern der Ärzte, die ja bei der Abrechnung mit den Kassen nicht bestimmte Krankheiten eingeben, sondern die zugeordnete Code-Bezeichnung. Diese könnte auch - bei entsprechender Organisation der elektronischen Patientenakten - sehr gut für eine Suche im Rahmen der vorliegenden Erfindung eingesetzt werden.

Wird in der elektronischen Patientenakte (EPR) des Patienten relevante Information gefunden, so erscheint in einem Teilbereich der grafischen Benutzeroberfläche am computerbasierten Arbeitsplatz des Arztes ein Stichwort, das die Information charakterisiert. Vorzugsweise ist mit dem Stichwort ein darunter verborgener "Link" verknüpft, sodass durch einen Click auf den Link die gesamte Information (Befund, Messwerte, medizinische Bilder) aus der EPR heruntergeladen und auf dem Arbeitsplatz sichtbar gemacht werden kann. In einer weiteren Ausgestaltung der Erfindung erscheint neben dem Stichwort auch das zugehörige Datum, an dem die gefundene Information erhoben wurde.

In einer weiteren vorteilhaften Ausgestaltung der Erfindung ist die Information in "vertrauliche" und "nicht vertrauliche" Information vorklassifiziert. Findet der kontextsensitive Browser als "vertraulich" gekennzeichnete Information, so erscheint am Bildschirm des behandelnden Arztes nur ein für sich noch nicht vertrauenskritischer Hinweis, evtl. nur das Datum des Ereignisses und das Schlüsselwort. Der Arzt weiß nun, dass ein evtl. entscheidungsrelevantes Ereignis in der Vergangenheit stattgefunden hat und kann mithilfe der am Bildschirm erschienen Hinweise den Patienten hierzu befragen. Dieser muss dann selbst entscheiden, ob er sich dazu äußern will.

Wie bereits weiter oben angedeutet wurde, kann erfindungsgemäß auch vorgesehen sein, dass nicht die gefundene Information selbst vollständig am Bildschirm angezeigt wird, sondern nur diese Information charakterisierende Teilinformationen, welche aus einer Teilmengen der insgesamt vorhandenen Informationen besteht. Diese Teilinformation kann beispielsweise sein: das gefundene Stichwort, der Zeitpunkt der Eintragung, die Organisation, von der die Information stammt, der Name dessen, der die Information eingetragen hat, zugeordnete Verwaltungscodes wie ICD oder DRG od. dgl.

Um die Gefahr eines Zugriffs Unbefugter im Rahmen einer erfindungsgemäßen Suche auszuschalten, kann in weiterer Ausgestaltung der Erfindung vorgesehen sein, dass die gefundene Information erst nach Anwendung eines Zugriffs-Schutzmechanismus, wie z. B. einer Passwort-Eingabe, freigegeben und dargestellt wird.

Bei besonders umfangreichen elektronischen Patientenakten oder wenn gleichzeitig auch eine Recherche in Archiven stattfindet, kann gemäß einer weiteren Ausgestaltung der Erfindung vorgesehen sein, dass die mit Hilfe der Stichwortsuche gefundene Information durch intelligente computerbasierte Expertensysteme dahingehend bewertet wird, ob diese für den aktuellen Untersuchungsprozess entscheidungsrelevant ist, und dass nur entscheidungsrelevante Information am Bildschirm dargestellt wird. Ein Beispiel hierfür ist, wenn zum Stichwort "Bauchschmerz" in der EPR ein aus einem lange zurückliegenden Unfall mit Schnittverletzungen am Bauch vorhandene Information gefunden wird. Diese würde das Expertensystem ausscheiden, da diese abgeheilte alte Bauchverletzung für jetzt gesuchte Ursachen von Bauchschmerzen sicher nicht in Frage kommen dürfte.

In vielen Fällen sind in der elektronischen Patientenakte oder in einem medizinischen Archiv medizinisch diagnostische Bilder enthalten, die nicht nach bestimmten Schlüsselwörtern abgespeichert sind, sodass sie bei einer solchen Suche gar nicht erfassbar sind. Um diese Schwierigkeit zu beseitigen ist in Ausgestaltung der Erfindung vorgesehen, dass angestoßen durch eine Suchanfrage mit bestimmten Schlüsselwörtern abgespeicherte medizinisch diagnostische Bilder in der elektronischen Patientenakte und/oder dem medizinischen Archiv mittels Bilderkennungsalgorithmen analysiert und daraus Schlüsselwörter zur Klassifizierung generiert werden, die mit den Schlüsselwörtern der Suchanfrage verglichen werden. Auf diese Art und Weise werden dem anfragenden Arzt nur solche Bilder geliefert bzw. Hinweise auf solche Bilder angezeigt, die wirklich im Zusammenhang mit der jeweiligen Krankheit des Patienten steht.

Beispiele für solche Bilderkennungs-Algorithmen sind 2D oder 3D Segmentierungs-Algorithmen, die Körperorgane an ihren Umrissen oder anderen geometrischen Merkmalen erkennen und die organspezifische Schlüsselwörter liefern können, wie z. B. "Herz-Aufnahme", "Gehirn-Aufnahme" od. dgl.

Für die erfindungsgemäße Klassifizierung eignet sich auf Computer Aided Diagnosis (CAD)-Algorithmen, die eine automatische Befundungs-Unterstützung vornehmen, wie z. B. bei der Erkennung von Tumoren, Knochenbrüchen od. dgl. Auch Algorithmen zur Texturanalyse können hier eingesetzt werden.

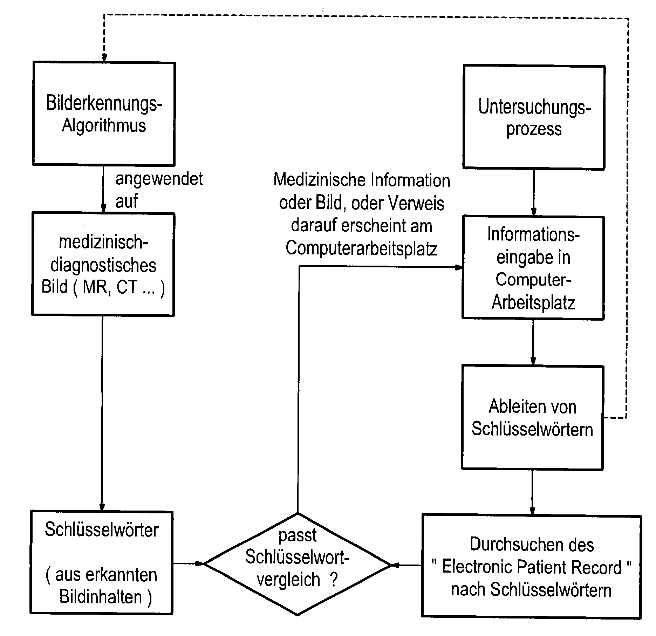

Weitere Vorteile, Merkmale und Einzelheiten der Erfindung ergeben sich aus der nachfolgenden Beschreibung eines Ausführungsbeispiels sowie anhand der Zeichnung, die ein Ablaufdiagramm des erfindungsgemäßen Systems in unterschiedlichen Varianten zeigt.

Auf der rechten Seite der Figur ist dargestellt, wie im Verlauf eines Untersuchungsprozesses der Arzt eine Informationseingabe an seinem computergestützten Arbeitsplatz vornimmt. Aus den eingegebenen Angaben werden selbsttätig, also ohne Zutun des Arztes, durch einen kontextbasierten intelligenten Algorithmus Schlüsselwörter abgeleitet und ebenfalls automatisch eine Suche in entweder der elektronischen Patientenakte EPR oder in einem medizinischen Archiv nach diesem Schlüsselwort bzw. diesen Schlüsselwörtern gestartet. Werden beim Schlüsselwortvergleich passende Informationen gefunden, so erscheint die medizinische Information oder ein Verweis auf diese Information oder ein Bild bzw. ein Verweis darauf am Computerarbeitsplatz.

Geht es darum, auch in der EPR abgespeicherte medizinisch-diagnostische Bilder zu erfassen, die - wie häufig üblich - nicht mit bestimmten Schlüsselwörtern abgespeichert sind, sodass sie bei der vorstehend beschriebenen Suche gar nicht erfasst werden können, so kann in Ausgestaltung der Erfindung nach dem Ableiten von Schlüsselwörtern am computergestützten Arbeitsplatz (wie es in der Figur links gezeigt ist) ein Bilderkennungs-Algorithmus auf medizinisch-diagnostische Bilder in der elektronischen Patientenakte oder auch in einem medizinischen Archiv angewendet werden, wobei dieser Bilderkennungs-Algorithmus wiederum Schlüsselwörter aus den analysierten Bildern ableitet. Entspricht dieses durch den Bilderkennungs-Algorithmus gewonnene Schlüsselwort dem Schlüsselwort der Suchanfrage, die vom computergestützten Arbeitsplatz selbsttätig gestartet worden ist, so erscheint wiederum das Bild oder ein Verweis darauf am Computerarbeitsplatz.

### Hierzu ein spezielles Beispiel:

Der Arzt trägt Information über eine gerade festgestellte Schwangerschaft einer Patientin in deren EPR ein. Der kontextbasierte intelligente Browser im Hintergrund greift ihm bekannte Schlüsselwörter wie "Schwangerschaft" (oder z. B. auch den zugehörigen ICD-Code) auf und durchsucht die Patientenakte nach früheren Schwangerschaften oder auch verwandten Ereignissen, wie einem Schwangerschaftsabbruch. Gefundene Information wird in einem Teilfenster sichtbar gemacht, gegebenenfalls zusammen mit dem zugehörigen Datum. Der Arzt entscheidet nun, ob ihm diese Information wichtig erscheint für den aktuellen Behandlungsprozess und kann die vollständige Information durch Markierung, Click, etc. abrufen. Ein intelligenter Algorithmus kann dabei die gefundene Information weiterfiltern, um nur auf entscheidungserhebliche Informationen zu verweisen. So werden im obigen Beispiel keine normal verlaufenden Schwangerschaften angezeigt, sondern nur solche mit aufgetretenen Komplikationen.

In einem anderen speziellen Beispiel trägt der Arzt den Namen eines verschriebenen Medikaments in die elektronische Patientenakte ein. Der Browser greift den Medikamentennamen , oder aus einer zugeordneten Datenbank die generische Substanz des Medikaments, auf, durchsucht die EPR, und meldet aus der Vergangenheit bekannte Unverträglichkeiten, Querempfindlichkeiten etc.

Als drittes Beispiel schließlich kann im Zusammenhang mit der oben als Beispiel angenommenen Schwangerschaft auch unter dem Stichwort "Schwangerschaft", "Gebärmutter" od. dgl. ein Schlüsselwort generiert und gleichzeitig ein Bilderkennungs-Algorithmus auf die nicht klassifizierten medizinischdiagnostischen Bilder in der EPR angewendet werden, um selbsttätig festzustellen, ob beispielsweise eine Gebärmutteraufnahme er Patientin vorliegt, die in Verbindung mit Informationen zu früheren Schwangerschaften oder Schwangerschaftsabbrüchen sehr entscheidungserhebliche Informationen für den momentan behandelnden Arzt liefern könnte.

## Patentansprüche

1. System zum Auffinden und Darstellen entscheidungsunterstützender Informationen in elektronischen Patientenakten oder medizinischen Archiven, **dadurch gekennzeichnet, dass** ein kontextbasierter intelligenter Algorithmus aus den Eingaben des Arztes an einem lokalen computerbasierten Arbeitsplatz selbsttätig Schlüsselwörter ableitet und Patientenakten und/oder Archive nach diesen Schlüsselwörtern durchsucht, und dass zu den Schlüsselwörtern relevante Informationen oder ein Link auf diese Informationen am lokalen computerbasierten Arbeitsplatz dargestellt werden.

2. System nach Anspruch 1, **dadurch gekennzeichnet, dass** mit Hilfe eines medizinischen Tesaurus aus einem, aus der Eingabe des Arztes abgeleiteten Stichwort mehrere verwandte Stichwörter abgeleitet und zusätzlich zur Suche verwendet werden.

3. System nach Anspruch 1 oder 2, **dadurch gekennzeichnet, dass** ein aus der Eingabe des Arztes abgeleitetes Stichwort mit Hilfe eines medizinischen Tesaurus in ein vordefiniertes verwandtes Suchwort übersetzt wird, um eine standardisierte Suche zu ermöglichen.

4. System nach einem der Ansprüche 1 bis 3, **dadurch gekennzeichnet, dass** das Suchstichwort einem Verwaltungscode des Gesundheitswesens, z. B. dem ICD-Code oder dem DRG-Code, zugeordnet wird und die Suche mit Hilfe dieses standardisierten Codes durchgeführt wird.

5. System nach einem der Ansprüche 1 bis 4, **dadurch gekennzeichnet, dass** ein kontextsensitiver Browser als "vertraulich" vorklassifizierte Informationen nur als Hinweis mit Schlüsselwort und Datum des Ereignisses am computerbasierten Arbeitsplatz anzeigt.

6. System nach Anspruch 5, **dadurch gekennzeichnet, dass** nicht die gefundenen Informationen selbst vollständig am Bildschirm angezeigt werden, sondern nur diese Informationen charakterisierende Teilinformationen, welche aus einer Teilmenge der Informationen bestehen.

7. System nach Anspruch 5 oder 6, **dadurch gekennzeichnet, dass** die gefundene Information erst nach Anwendung eines Zugriffs-Schutzmechanismus, wie z. B. einer Passwort-Eingabe, freigegeben und dargestellt wird.

8. System nach einem der Ansprüche 1 bis 7, **dadurch gekennzeichnet, dass** die mit Hilfe der Stichwortsuche gefundene Information durch intelligente computerbasierte Expertensysteme dahingehend bewertet wird, ob diese für den aktuellen Untersuchungsprozess entscheidungsrelevant ist, und nur entscheidungsrelevante Information am Bildschirm dargestellt wird.

9. System nach einem der Ansprüche 1 bis 8, **dadurch gekennzeichnet, dass**, angestoßen durch eine Suchanfrage mit bestimmten Schlüsselwörtern, abgespeicherte medizinisch diagnostische Bilder in der elektronischen Patientenentenakte und/oder dem medizinischen Archiv mittels Bilderkennungs-Algorithmen analysiert und daraus Schlüsselwörter zur Klassifizierung generiert werden, die mit den Schlüsselwörtern der Suchanfrage verglichen werden.

10. System nach Anspruch 9, **gekennzeichnet durch** 2D oder 3D Segmentierungs-Algorithmen die Körperorgane (an ihren Umrissen oder anderen geometrischen Merkmalen) erkennen und organspezifische Schlüsselwörter liefern (z. B. "Herz-Aufnahme", "Gehirn-Aufnahme").

11. System nach Anspruch 9 oder 10, **gekennzeichnet durch** die Verwendung von Computer Aided Diagnosis (CAD)-Algorithmen, die eine automatische Befundungs-Unterstützung vornehmen.
